# EUROPEAN PATENT APPLICATION

(11) **EP 1 134 002 A2**
(43) Date of publication of application: **19.09.2001**
(21) Application number: 01810085.9
(22) Date of filing: 30.01.2001
(51) Int. Cl.: A61N 1/30

(54) **Apparatus for realising a process of sterilisation using iontophoresis**

(30) Priority: 16.03.2000 CH 4992000
(71) Applicant: Barston International Ltd., 1211 Genève (CH)
(72) Inventor:
(74) Representative: Gaggini, Carlo, Dipl.Ing.

(57) **Abstract**

The apparatus comprises an electric circuit with at least a voltage generator (7) that delivers an electric direct current that distinguishes the apparatus from devices of similar type known according to the state of the art that operate using an alternating current in contrast. Furthermore the circuit of the apparatus comprises a device (5) which maintains the electric current constant independently of the variations in the impedance Z of the circuit, the impedance Z depending on the portion of the skin tissue of the zone of the body of the patient via which the current passes. The advantages of the inventive apparatus in comparison to the ones known thus far are the shorter time duration of the ionophoresis application at lower voltages for obtaining the same results and the better control of the apparatus the operator can exert owing to the elimination of the factor of uncertainty due to the variations in the impedance Z of the circuit caused e.g. to local sweating of the patient.

## Description

The present invention concerns an apparatus of sterilisation, using ionophoresis, of the ducts of a tooth infected by pathogens as described in the introductory portion of the claim 1.

The principle of treatment of infected teeth using ionophoresis is known from the specialist literature, in particular from the works of P.D. Bernard (compare e.g. the publication titled "Rapid and Radical Treatment of the Pulpa Tissues and Periapical Affections Using the New Methods of lonophoresis and of Diadynamic Therapy" (II trattamento rapido e radicale delle gengive pulpari e delle affezioni periapicali mediante I nuovi metodi di ionoforesi e die terapia diadinamica) published and edited by the Physiotechnie SA (Company), Paris, 1929, as well as by Ph. Lagarde and R.P. Lagarde who published, not dated but certainly after 1974, a study "Treatment of Infected Teeth" (Trattamento dei denti infetti), published in France by the Imprimeriex Company, 51bis Av. de Pessicart, Nice. In this publication a large bibliography is cited comprising 40 titles concerning various methods of treating dental infections among which also the method using ionophoresis is cited. In the publications cited also devices, or apparatuses respectively, are illustrated which are suitable for realisation of the ionophore treatment: their function, however, is not described other than in some general indications which do not permit gaining anything other than a general knowledge concerning the formation of "OH" ions penetrating the "aberrant" ducts of the teeth.

More detailed information can be found fortunately in the patent literature concerning devices as described in the studies cited

In particular the FR-916843 describes an improvement of the devices used in medical or dentistry electrotherapy in which a circuit is supplied from the source of direct current the output current of which is regulated using two potentiometers. To the direct current a source of low voltage current is superimposed the voltage of which can be regulated using another potentiometer which, more specifically, is pre-set not in function of the voltage it supplies but rather in such a manner that its index gives the cursor position required for obtaining the optimum analgetic effect in function of the intensity of the direct current supplied. In other words, according to this document, to the direct current applied an alternating current is superimposed as "a low frequency sine differential in potential of 5 volts provides an anaesthetic action if it is superimposed to an externally supplied current of 1 milliamp, but if this same 5 volt ac voltage is applied together with a direct current of 3 or 4 milliamps, its action onto the sensitivity predominates, where this action becomes intolerable with a direct current intensity of 5 milliamps".

Expressed in other words this patent cited teaches that the use of an electrotherapeutic dentistry treatment be used for obtaining an anaesthetic effect, which essentially differs from the objective of the present invention which proposes a method of sterilisation (and thus of disinfection) of the ducts of the tooth treated using ionophoresis. Furthermore it is to be stated here that for obtaining the desired effect application of a dc current to which an ac current is superimposed as (compare the initial paragraph of the description) that "the dc current in some cases is tolerated badly". From this document thus no teachings can be gained concerning the use of ionophoresis as a therapeutic method and no useful information whatsoever can be obtained for a device suitable for implementing a therapeutic method of the type mentioned.

In CH-257243 an electrotherapeutic device is described permitting generation of a modulated current of a frequency of a plurality of seconds as desired and a syncope current of short periods. Also in this case it is proposed for obtaining a desired therapeutic effect (e.g. pain, atonicity and the diagnosis of neuro-muscular disorders) a modulated current be applied which is generated by the device with the help of thermoionic valves co-operating with interrupt relays according to the technological state of the art at the time of the deposition of the invention cited. Also in this case we are far away from the objective of the present invention and thus it is ensured that the technique of therapeutic treatment described there definitely contrasts the objective of the present findings. The devices described in the documents cited, in particular in the cited study by Ph. And R.P. Lagarde, page 29, propose use of two poles namely a negative electrode and a positive electrode. The negative electrode is formed by a metallic probe which is shaped as a needle as it is to penetrate into the depth of a duct of the tooth to be treated, whereas the other pole, or the positive electrode respectively, is brought into direct contact with a body zone of the patient and preferentially is shaped as a metallic cylinder which the patient holds in his hand.

This working method as well as the form of the poles thus is known from the literature and from practical use and constitutes the state of the art on which the present invention is based.

The use of an alternating current is applied in the devices known, as with the electrophoretic application predominantly generation of an anaesthetizing effect was aimed at rather than a sterilizing effect proper. For obtaining an anaesthetizing effect it is necessary indeed to make sure, as it has been proven in practical application, that after a positive current impulse a negative impulse follows, as the anaesthetizing effect is based on the decoding of the nerve ends. This mechanism of anaesthesia in practical application does not yield satisfactory results however, and for this reason today the anaesthetizing effect of the ionophoretic treatment preferentially is renounced and the sterilizing effect of the through-passing current primarily is relied on.

For this reason the present invention provides application of a direct current rather than of an alternating current, which entirely differs from the previous practice and teachings. For obtaining the desired disinfection effect it is sufficient to apply a direct current which presents the advantage also that it can be metered more easily in its timing. Furthermore, the disinfecting effects being equal, a direct current can be applied at lower voltage and over shorter time periods than an alternating current resulting in a reduction of the dangers and of the total application time.

It thus is the objective of the present invention to realise an apparatus for sterilising a tooth using ionophoresis and provided with optimum characteristics for obtaining the sterilising effect within the shortest possible time and at to eliminate the disadvantages of the known devices. In particular it has been found that in the known devices the electric current applied varied together with variations in the circuit impedance Z. Such variation in the impedance in a circuit in which one of the poles directly contacts the skin of the patient can have various causes the main one being the variation in humidity of the skin tissue caused by sweating, etc.

These variations in the impedance of the circuit, caused frequently by physiologic effects uncontrollable nor foreseeable by the operator, greatly impair the constancy of the current applied and thus render control of the working process difficult and thus endanger the good result of the treatment. The present invention also aims at solving this problem and thus at rendering the treatment of the local physiologic conditions of the patient.

These objectives are met using an apparatus according to the introductory portion of the claim 1 presenting the characteristics according to the characterising portion of the claim 1.

According to these characteristics the electric current applied is a direct current - generated by an adjustable generator which normally supplies a current at a maximum voltage of 89 volts - and furthermore the electric circuit comprises a device which maintains the direct current constant as the impedance Z of the circuit varies which takes into account that the impedance Z essentially is determined by the skin tissue of the body zone of the patient - normally a hand - via which the current passes.

The advantages of the inventive apparatus are the speed of the treatment, the reduction of risks owing to the reduced voltage of the current applied and the possibility of strictly controlling the treatment applied freeing it from the influences of uncontrollable parameters such as sweat secretion of the patient, the degree of humidity of the air in the surrounding room, etc.

Further advantages can be realised owing to a series of characteristics which form the objects of the dependent claims and which will be described in more detail in the course of the description of some examples of realisation of the present invention. In particular it will be described e.g. in which manner the inventive apparatus is to be laid out for applying the treatment of ionophoretic disinfection of the tooth depending on the position of the tooth in the dental arc and on the pathology of the tooth. According to a preferred variant of realisation of the present invention the apparatus is to comprise a programmable micro processor using which it is possible to monitor and to control all functions of the apparatus.

These and other characteristics of the present invention will be described in more detail with reference to several examples of realisation illustrated in the corresponding Figures.

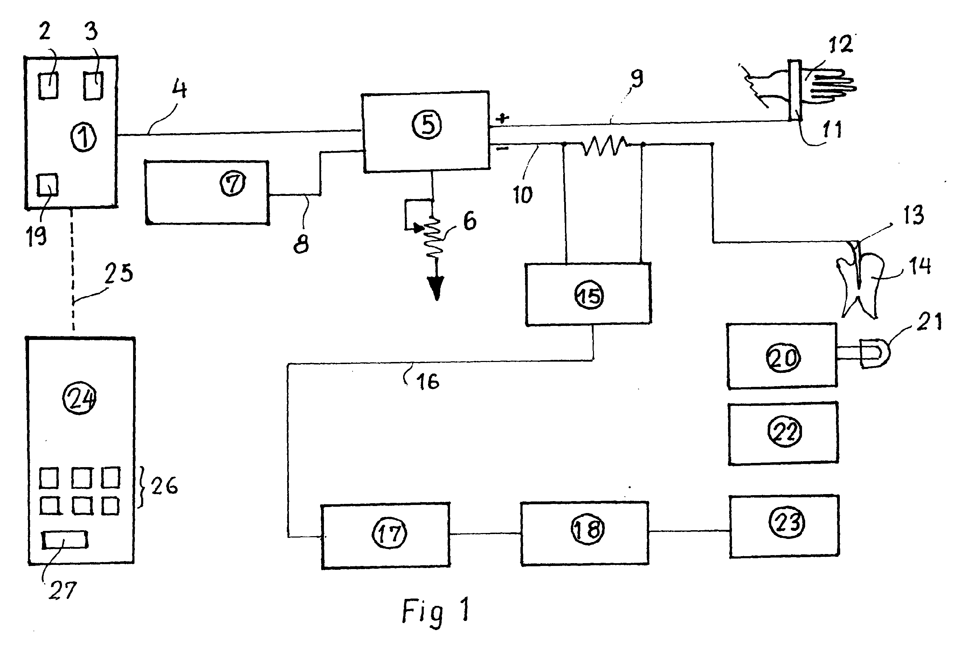

In the Fig. 1 a block diagram is shown with several variants of an inventive apparatus, whereas the table 1 contains, in the sense of mere examples, indications concerning the interdependence between the pathology of the tooth to be treated and the amount of electric current to be applied. Already here it is to be stressed that these indications are mere indications in so far as it is a specific characteristic of a dependent claim that the intensity of the current to be applied depends on the time duration of the application for each individual sterilising treatment in the sense that the product of two quantities must take into account, in addition of the pathology of the tooth to be treated, also of the position of the tooth within the dental arc. This interdependence, however, is determined using the specific experience of the operator and which thus exceeds the patent protection claimed in the present application.

In the Fig. 1 thus a block diagram is shown of an apparatus for implementing the present invention.

In this diagram a box designated 1 indicates a manual control panel provided with at least one push button 2 for starting the apparatus (start) and a push button 3 for stopping the apparatus (stop). The control panel is connected via a circuit line 4 with a device delivering constant direct current 5 the circuit of which is provided for adjusting the current as set on the regulator 6 by the operator and which is maintained constant over time even if the contacting points (electrode held in the hand of the patient, and the needle respectively, in the root duct of the tooth, as will be explained in the following) change the physical characteristics of conductivity. The device delivering constant current 5 is supplied with direct current by a voltage generator 7- supplied from the grid or by a battery - the function of which is to generate a voltage of not more than 80 volts at the maximum (for safety reasons prescribed by the law) and to exert control that the maximum limit of 5 mA is never exceeded. The voltage generator supplies the constant current device 5 via a circuit line 8. From the constant current device 5 two circuit lines 9 and 10 extend. The circuit 9 corresponds to the positive pole and extends to a handle 11 which the patient presses in his hand 12. Of course this solution just gives an indication as instead of a handle any other element providing direct contact with the patient, in particular between a zone of the skin and the circuit 9, could be provided.

The other circuit 10 (which corresponds to the negative pole) extends to a needle 13 which is inserted for the treatment in a cavity which has been previously prepared in the tooth 14 in such a manner that the process of disinfection using ionophoresis of the ducts of the tooth 14 as described in detail in the literature cited in the introduction above can be effected.

In the line 10 a current sensor 15 is inserted the function of which is to ensure continuity of the electric circuit by emitting an electric signal via the line 16 to a detector which scans the on/off state of the circuit. This later in turn controls the current integrator 18 which continually effects the multiplication of the intensity of the current applied, measured in mA with the duration of the application time according to a preferred variant of realisation of the present invention. On the control panel 1 a timer 19 is provided using which, according to a preferred variant of realisation of the present invention, the time duration of the application of current for each single treatment of sterilisation of the tooth can be pre-set by the operator. In the simplest lay-out of the inventive apparatus the operator determines these settings on the basis of his personal experiences and/or with the help of the tables indicating the intensity of the current to be applied (compare e.g. the enclosed table 1) in function of the type of pathology of the tooth to be treated and taking into account also the position of the tooth in the dental arc. Also for this operation he can make use of experimentally established nomograms that, however, are not object of the present invention.

The electric system furthermore can comprise a detector 20 of threshold current intensity (set e.g. at 4 mA) with a LED 21 indicating that the maximum current intensity (5 mA) is almost reached, excess over which pre-set maximum current intensity (5 mA as a rule) is to be avoided in any case, as well as an Amp meter 22 and a Coulomb meter measuring the current intensity in mA/sec (which elements are not indispensable for realising the present invention). These additional elements represent precautionary safety elements that increase the security of the treatment but none of them adds anything to the present invention itself.

In the block diagram shown in the Fig. 1 a programmable microprocessor 24 is indicated which, according to a preferred embodiment of the present invention, permits monitoring and control of all the functions of the inventive apparatus which essentially can replace the control panel 1 to which, as seen in the Fig. 1, it connected via a line 25 indicated in dashed lines (which signifies that it represents an alternative solution).

The programmable microprocessor 24 is provided, according to a first preferred embodiment of the present invention, in particular for monitoring and controlling the maximum voltage of the current generated by the current generator 7 (80 Volts), the device 5 for maintaining the electric current constant while the impedance Z of the circuit varies (due to e.g. the variation in electric conductivity between the hand 12 of the patient and the handle 11) as well as the product of the intensity of the current applied multiplied with the time duration of application in function of the position of the tooth to be treated in the dental arc and of the pathology to be treated.

In this context it is stated that according to a preferred embodiment of the present invention for the success of the disinfection treatment of the tooth using ionophoresis the time duration of the application of the electric current preferentially is determined taking into account that the product of the regulated intensity of the current, measured in mA, with the duration of application, measured in seconds, depends, if optimum results are to be obtained, on the position in the dental arc of the tooth to be treated and on the pathology of the tooth itself. These relations are based on empiric observations gained from a great number of treatments and are not part of the present invention the aim of which merely is to provide the expert with an apparatus for realising optimum treatments using ionophoresis.

The programmable microprocessor 24 of course is provided with a keyboard 26 using which all data required for realising any specific type of treatment using ionophoresis can be fed in and thus applied in programming.

According to a further preferred embodiment of the present invention the microprocessor e.g. is provided with a safety arrangement (schematically indicated with the reference sign 27 in the Fig. 1 showing the block diagram) which allows the start of the desired treatment using ionophoresis only after confirmation by the operator that the tooth to be treated has been subject to a satisfactory anaesthesia which the operator checks by applying a suitable pain test. The purpose of this arrangement is evident: the treatment of the ducts of a tooth using ionophoresis can be painful if the tooth has not been properly anaesthetised previously. For this operation preferentially chemical anaesthesia is applied via injection to the ducts. It is to be noted here that neither the operation of preparing the dental ducts (opening by trepanning, elimination of the nerve) nor the anaesthesia in no way constitute any obstacle to the application of the inventive apparatus.

In another preferred embodiment of the present invention the safety arrangement 27 is provided with a device (not shown) emitting an electric test discharge that can be regulated by the operator who thus can make sure that the tooth to be treated has been subject to an anaesthesia that satisfactory for the predetermined conditions for the ionophoresis treatment planned. Owing to this additional arrangement the operator, after having prepared the duct of the tooth, having applied a chemical anaesthesia by injection, and having extracted the nerve, thus easily can make sure without loosing time that the anaesthesia still is effective to such extent that for the patient the treatment of sterilisation using ionophoresis to which he will be subject subsequently will be painless which treatment can be of a duration of several minutes and longer.

According to another preferred embodiment of the present invention the quantity of electric direct current supplied by the voltage generator 7 during a treatment of sterilisation using ionophoresis, measured in mAsec, ranges between 100 and 1000 m/Asec, and more precisely between 200 and 500 mA/sec. This indication serves for laying out the dimensions of the electric lines and circuits according to the block diagram of the apparatus.

The block diagram described here with reference to the Fig. 1 is not the only solution imaginable for realising the inventive apparatus for which in its most general form it is sufficient if a voltage generator 7 is provided which supplies a continuous voltage, and a device 5 which maintains the electric direct current constant independently of the varying impedance Z of the electric circuit. The other devices described serve for facilitating the task of the operator and in particular for enhancing the operating safety and for avoiding any danger of occurrence of pain in the patient due to a ionophoresis treatment of the dental ducts which is excessively intense.

The secondary electric circuits forming the various components of the block diagram 1 such as the programmable microprocessor 24 are well known to the specialist in the field and mostly consist of elements readily available in the market. Thus a further description of the electric circuits of the individual components and a more detailed discussion of the electric circuit of the inventive apparatus can be dispensed with.

For the application of the inventive apparatus however, the application nomograms are most important which contain the relations between the position of the teeth in the dental arc, the pathology to be treated and the intensity of the ionophoresis treatment required for obtaining the desired results. The table 1 represents an example of the relation between the pathology of the tooth and the quantity of direct current to be applied. Of course the operator using the inventive apparatus must have similar information at his disposal based on his own personal experience or on the experiences of other specialists in order to make best use of the inventive apparatus. Such information to a great extent can be programmed, if a microprocessor 24 is incorporated, and thus always is at the disposal of the operator who only has to select the general parameters (tooth to be treated, pathology) for safely using the inventive apparatus. This presents the possibility of adopting a so-called expert system on the microprocessor 24.

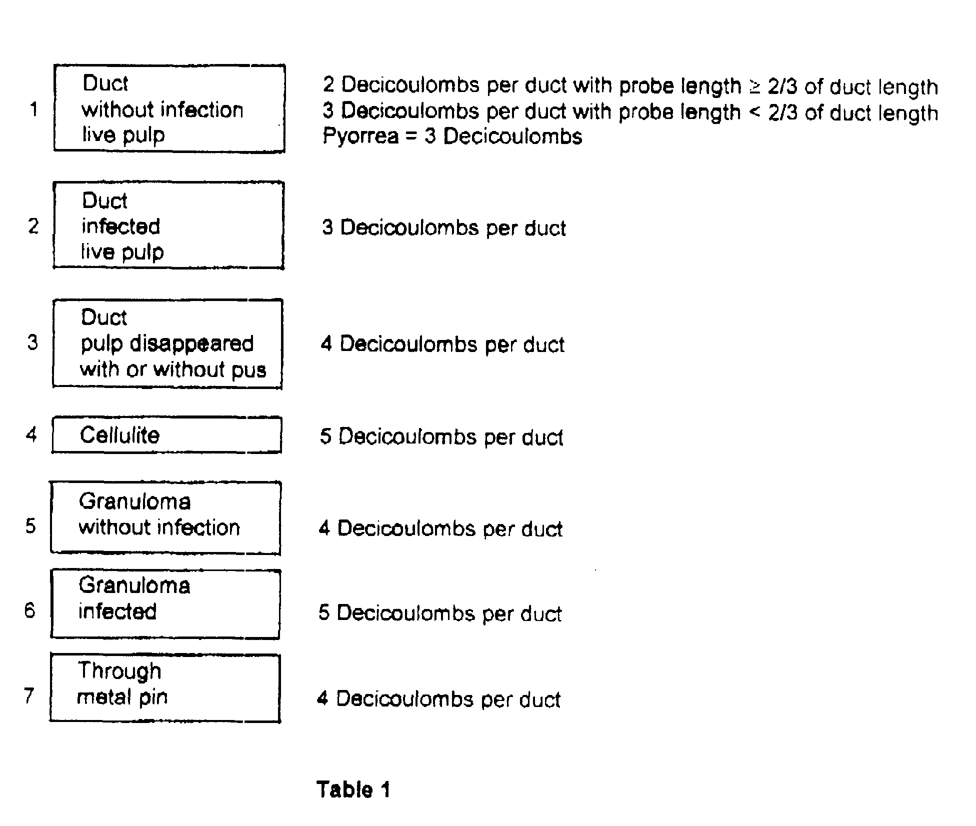

### List of the Elements Referred to in the Figures

- 1: manually operated control panel
- 2: start button
- 3: stop button
- 4: electric connecting line
- 5: device maintaining the current constant
- 6: controlling device
- 7: voltage generator
- 8: electric connecting line
- 9: electric connecting line
- 10: electric connecting line
- 11: handle
- 12: hand
- 13: needle
- 14: tooth
- 15: current sensor
- 16: electric connecting line
- 17: detector of on/off state of the circuit
- 18: current integrator
- 19: timer
- 20: detector for the threshold intensity of the current
- 21: LED (light emitting diode)
- 22: Amp meter
- 23: Coulomb meter
- 24: programmable microprocessor
- 25: electric connecting line
- 26: keyboard
- 27: safety arrangement

## Claims

1. Apparatus for realising a process of sterilisation using ionophoresis of the ducts of a tooth affected by pathogens, comprising an electric circuit with at least one adjustable electric voltage generator (7) and an applicator device supplied with electric current and presenting two poles, one of which is formed essentially as a needle (13) which can penetrate into the depth of a duct of the tooth whereas the other pole (11) is brought into direct contact with a zone of the body of the patient,
**characterized in that**
the voltage generator (7) delivers an electric direct current and that the circuit furthermore comprises a device (5) for maintaining the electric direct current constant while the impedance Z of the circuit varies, the impedance Z being determined by the zone of the body of the patient via which the current passes.

2. Apparatus according to the claim 1,
**characterized in that**
the electric voltage generator (7) generates a current voltage of 80 volts or less and is equipped with a device limiting the current intensity of the current delivered in such a manner that the intensity of the current at all times is kept below 5 mA.

3. Apparatus according to the claim 1,
**characterized in that**
the electric circuit comprises a timer (19) using which it is possible to pre-set the duration of the application of electrical current for each individual treatment of sterilisation, this duration being determined taking into account the requirement that the product of the intensity of the current applied, measured in mA, with the time duration of the application, measured in seconds, depends on the position in the dental arc of the tooth to be treated as well as on the pathology of this tooth.

4. Apparatus according to the claim 3,
**characterized in that**
the electric circuit comprises a control device (6) for the intensity of the electric current which can be set manually by the operator and a timer (19) which can be set manually by the operator and using which the operator pre-sets the intensity of the current to be applied and the time duration of the application, i.e. the product of these two values, based on nomograms wherein the position in the dental arc of the tooth to be treated, the pathology of the tooth to be treated and the corresponding value of the product of the intensity of the current with the time duration of the treatment are indicated.

5. Apparatus according to the claims 1 through 4,
**characterized in that**
the electric circuit comprises a programmable microprocessor (24) for monitoring and controlling all functions of the apparatus, and in particular
- the maximum voltage of the current generated by the voltage generator (7) (80 volt),
- the device (5) for maintaining the electric direct current constant while the impedance Z of the circuit varies,
- the product of the intensity of the current applied with the time duration of the application in function of the position in the dental arc of the tooth to be treated and of the pathology to be treated.

6. Apparatus according to the claim 5,
**characterized in that**
the microprocessor (24) furthermore is provided with a safety arrangement (27) that permits start of the ionophoresis treatment intended only after confirmation by the operator that the tooth to be treated has been subject to a satisfactory anaesthesia that the operator is to check by effecting a suitable pain test.

7. Apparatus according to the claim 5,
**characterized in that**
the safety arrangement (27) is equipped with an electric test discharge emitter device which can be adjusted by the operator who thus can make sure that the tooth to be treated has been subject to a proper anaesthesia satisfying the conditions provided for the ionophoresis treatment intended.

8. Apparatus according to the claim 8,
**characterized in that**
the quantity of constant electric current delivered by the voltage generator (7) during a sterilisation treatment using ionophoresis, measured in mA/sec, ranges between 100 and 1000 mA, and preferably between 200 and 500 mA/sec.
